# EUROPÄISCHE PATENTSCHRIFT

(11) **EP 2 453 880 B1**
(45) Veröffentlichungstag und Bekanntmachung des Hinweises auf die Patenterteilung: **13.02.2019**
(21) Anmeldenummer: 10734458.2
(22) Anmeldetag: 16.07.2010
(51) Int. Cl.: A61K 9/50, A61K 31/381

(54) **TRENNSCHICHTEN FÜR PHARMAZEUTISCHE ZUBEREITUNGEN ZUR VERHINDERUNG VON WECHSELWIRKUNGEN ZWISCHEN ARZNEISTOFFEN UND PHARMAZEUTISCH-TECHNOLOGISCHEN HILFSSTOFFEN**
SEPARATING LAYERS FOR PHARMACEUTICAL PREPARATIONS TO PREVENT INTERACTIONS BETWEEN MEDICINAL DRUGS AND PHARMACEUTICAL-TECHNOLOGICAL ADJUVANTS
COUCHES DE SÉPARATION POUR PRÉPARATIONS PHARMACEUTIQUES, DESTINÉES À EMPÊCHER LES INTERACTIONS ENTRE LES MÉDICAMENTS ET LES AGENTS AUXILIAIRES PHARMACEUTIQUES ET TECHNOLOGIQUES

(30) Priorität: 17.07.2009 DE 102009033621
(43) Veröffentlichungstag der Anmeldung: 23.05.2012
(73) Patentinhaber: ADD Technologies Ltd., 4133 Pratteln (CH)
(72) Erfinder: SCHLUETERMANN, Burkhard, 79280 Au (DE); GERBER, Frédéric, 68730 Blotzheim (FR); PRASCH, Armin, 79249 Merzhausen (DE)
(74) Vertreter: Henkel, Breuer & Partner
(86) Internationale Anmeldenummer: PCT/EP2010/004362
(87) Internationale Veröffentlichungsnummer: WO 2011/006670

(56) Entgegenhaltungen:
- WO-A2-2007/034503
- WO-A2-2007/122478
- WO-A2-2007/139886
- WO-A2-2008/077939
- WO-A2-2009/010238
- US-A- 5 508 276
- US-A1- 2008 226 711

## Beschreibung

Arzneistoffe sind bioaktive Substanzen, die in pharmazeutische Darreichungsformen integriert, als Arzneimittel appliziert werden, um die Funktionen des Körpers zu beeinflussen und Krankheitsbilder abzuschwächen, Krankheiten vorzubeugen oder diese zu heilen. Wie biologisch aktiv ein Wirkstoff ist, hängt u.a. von den strukturellen Eigenschaften des Arzneistoffs, seinen elektronischen Eigenschaften sowie der Hydrophilie und Lipophilie des Arzneistoffmoleküls ab. Unter strukturellen Eigenschaften wird hier nicht nur die gegenseitige Verknüpfung der einzelnen Atome eines Moleküls verstanden, sondern auch welche stereochemischen Strukturen dem Molekülaufbau zugrundeliegen, also dessen Konstitution, Konfiguration sowie Konformation, und wie diese mit den entsprechenden Rezeptoren des biologischen Systems in Wechselwirkung treten können [1].

Im Hinblick auf die elektronischen Eigenschaften eines Arzneistoffs stellen viele Pharmaka schwache Säuren oder schwache Basen dar. Im Falle der Säuren handelt es sich in der Regel um Carbonsäuren, Phenole oder NH-azide Verbindungen. Bei den Basen liegen meist Stickstoff-Basen vor. Nach Applikation einer Arzneiform liegen derartige Wirkstoffe, in Abhängigkeit von der Umgebung des Arzneistoffs, in protonierter oder nicht-protonierter Form vor, bzw. zwischen ionisierter und nicht-ionisierter Form des Arzneistoffmoleküls. Das Verhältnis von ionisierter zu nicht-ionisierter Form wird durch die Dissoziationskonstante K bzw. durch den pKa-Wert der Substanz beschrieben. Den quantitativen Zusammenhang zwischen ionisierter und nicht-ionisierter Form und dem pH-Wert der Umgebung liefert die Henderson-Hasselbach Gleichung. Ionisierte und nicht-ionisierte Form eines Arzneistoffs zeigen in der Regel ein gegenläufiges Löslichkeits- und Resorptionsverhalten [1,2].

In der nicht-ionisierten Form hat ein Arzneistoff in der Regel lipophilere Eigenschaften, die ionisierten, anionischen oder kationischen, Formen, zeigen häufig eine erhöhte Wasserlöslichkeit. Der Dissoziationsgrad diktiert das Resorptions- und Verteilungsverhalten des Arzneistoffs und somit die Wechselwirkung mit möglichen Rezeptoren in biologischen Systemen. Demzufolge kommt dem pH-Wert der Umgebung und dem pKa-Wert der Substanz eine große Bedeutung zu.

In biologischen Systemen wie z.B. beim Menschen bestimmen, bei oral applizierten Pharmaka, die pH-Verhältnisse im gesamten Verdauungstrakt, d.h. von der Mundhöhle bis ins Rektum, sowie der pKa-Wert der zu applizierenden Wirkstoffe über den biologischen Effekt des Arzneistoffs [2,3]. Neben der Dissoziation eines Arzneistoffs spielt die Lipophilie bzw. Hydrophilie des Arzneistoffmoleküls zur Wechselwirkung mit Rezeptoren des biologischen Systems noch eine wichtige Rolle. Hier hat sich der Lipid-Wasser-Verteilungskoeffizient P als Maß für die Lipophilie von Wirkstoffmolekülen eingebürgert. Zur *in vitro* Simulation geht man vom System Oktanol/Wasser bzw. Oktanol/Pufferlösungen aus.

Lipophile Stoffe haben einen hohen Verteilungskoeffizienten. Ähnlich den pKa-Werten beschreiben die P-Werte bzw. deren dekadischer Logarithmus, log-P-Werte, Eigenschaften des Gesamtmoleküls. pKa- und log-P-Werte stellen somit Wechselwirkungsparameter mit dem umgebenden System dar [2].

Die Diffusionsgeschwindigkeit von Pharmaka durch lipophile Membranen ist umso größer, je höher der Verteilungskoeffizient des Arzneistoffs ist.

Diese Betrachtungen zeigen, dass Arzneistoffe während der Passage durch den Verdauungstrakt vielfältigen Wechselwirkungen mit der Umgebung unterliegen, die alle einen Einfluss auf die Resorption und damit auch auf die Wirkung des Arzneimittels haben können.

Ziel sollte somit sein, den Arzneistoff optimal verfügbar zu machen, um eine vollständige Freisetzung des Wirkstoffs aus der Arzneiform zu gewährleisten.

Da Arzneistoffe in der Regel mit weiteren Hilfsstoffen kombiniert zu Arzneimitteln verarbeitet werden, sind neben der obigen Betrachtung auch formulierungsspezifische Aspekte bei der Hilfsstoffauswahl für eine zu entwickelnde Arzneiform zu berücksichtigen.

Neben den hier beschriebenen potentiellen Wechselwirkungen mit den physiologischen Bestandteilen innerhalb des gesamten Verdauungstraktes, können auch die pharmazeutisch-technologischen Hilfsstoffe, die zur Formulierung des Arzneimittels eingesetzt werden, Reaktionen und Wechselwirkungen mit den Bestandteilen des Verdauungstraktes eingehen, aber auch selbst mit den Wirkstoffen selbst interagieren.

Im Hinblick auf die oben beschriebenen Änderungen, die ein Arzneistoffmolekül bei der Passage durch den Verdauungstrakt erfahren kann, kann sich auch ein pharmazeutischer Hilfsstoff ändern. Somit beginnen eine Reihe von pharmazeutischen Hilfsstoffen bei Zutritt von Flüssigkeit zu quellen bzw. sich aufzulösen. Diese Quell- und Lösungsvorgänge führen zu bzw. induzieren bekanntermaßen bei Tabletten den Zerfall oder bei Hartkapseln das Auflösen der Kapselhülle.

Flüssigkeiten, Flüssigkeitsvolumina und pH-Wert der Säfte im Verdauungstrakt sind sehr unterschiedlich zusammengesetzt und unterliegen vielfachen Einflussfaktoren und müssen demzufolge bei der Formulierung eines Arzneimittels beachtet werden. Nahrungsaufnahme, flüssig und/oder fest, verändert das Profil zusätzlich.

Um Wechselwirkungen mit den Flüssigkeiten des Verdauungstraktes zu unterbinden werden Wirkstoffe, wirkstoffhaltige Granulate oder Pellets bzw. ganze Darreichungsformen, wie Tabletten oder Kapseln, mit Überzügen versehen. Diese Überzüge haben eine Schutzfunktion und können sehr komplex zusammengesetzt sein. In der Regel handelt es sich bei diesen Überzugsmaterialien um anorganische oder organische Substanzen bzw. Substanzgemische [4]. Neben den Überzugmaterialien selbst werden Hilfsstoffe wie Weichmacher, Antiklebestoffe, Porenbildner, Farbstoffe, Geschmacksstoffe etc. zugesetzt [5].

Darüber hinaus können mit makromolekularen Carbonsäuren beschichtete Wirkstoffe, Zwischenprodukte oder Darreichungsformen den Transport des Wirkstoffs in tiefere Darmabschnitte gewährleisten, wo der Arzneistoff freigesetzt, möglicherweise resorbiert wird oder lokal appliziert werden kann. Weiterhin können makromolekulare Carbonsäuren u.a. auch zur pH-abhängigen Reduzierung der Auflösungsgeschwindigkeit eingesetzt werden.

Bei den organischen Überzugsmaterialien werden in der Regel polymere Filmbildner eingesetzt. Wie bei den Arzneistoffen gibt es bei den polymeren Filmbildnern ebenfalls Wechselwirkungen zu beachten, insbesondere wenn es sich um Polymere handelt, die ihren Charakter im Lauf der Passage durch den Verdauungstrakt ändern können. So können polymere Carbonsäuren bei Erreichen des Dünndarms durch das geänderte pH-Milieu des Darms gegenüber dem Magen deprotoniert werden und liegen dann als anionisches Makromolekül vor. Dabei gehen diese im Rahmen einer Anlösephase, die in der Regel einen hochviskosen klebrigen Zustand darstellt, sukzessive vollständig in Lösung. Dabei werden Stadien unterschiedlicher Viskosität durchlaufen. Im Grenzbereich zur Löslichkeit solcher Filme bilden sich sehr viskose, klebrige Massen aus. Bei Vorliegen von Gegenionen, wie z.B. Arzneistoffkationen, können unter Umständen sehr schwer lösliche, hochklebrige Massen, die polymere, salzartige Verbindungen enthalten können, entstehen.

Die An- und Auflösung dieser polymeren Carbonsäuren ist aber von vielen Faktoren abhängig. Neben der Löslichkeit des zugrundeliegenden Makromoleküls und der Dissoziationskonstanten der Säurefunktionen sind unter anderem auch die Anzahl der vorhandenen Säuregruppen des Polymermoleküls als potentiell kritisch einzustufen. Eine erhöhte Anzahl funktioneller Gruppen im Polymermolekül der Polycarbonsäure bedeutet in der Regel eine Zunahme der Interaktionsmöglichkeiten. Darüber hinaus spielt die lonenkonzentration eine wichtige Rolle, die bei An- und Auflösung durch das Auflösungsmedium, das mit der Darreichungsform in Kontakt tritt, erreicht wird. Eine höhere lonenkonzentration des Mediums bedeutet in der Regel ein verzögertes Auflösungsverhalten. Alterungsphänomene des Polymerfilms können sich in der Regel auch negativ, dass heißt in diesem Fall freisetzungsverzögernd, auf die Filmeigenschaften auswirken.

Bedeutsam ist auch der Effekt der anderen Formulierungsbestandteile eines Polymerfilms wie z.B. Weichmacher, Porenbildner, Antiklebemittel und Farbstoffe etc. Diese haben in der Regel auch einen Einfluss auf das Auflösungsverhalten des Films. Darüber hinaus wird An- und Auflösung auch durch das pH-Milieu der benachbarten Schichten des polymeren Carbonsäurefilms beeinflusst. Basische Komponenten können bei polymeren Carbonsäuren zu erhöhten Lösungsgeschwindigkeiten beitragen, saure Verbindungen zu einer Verzögerung der Auflösung.

Die Interaktionen zwischen Bestandteilen eines zu überziehenden Pelletkerns und den polymeren Carbonsäuren haben hauptsächlich physikalische bzw. physikalisch-chemische Ursachen und sind u.a. auf elektronische Wechselwirkungen wie Oxidation, Reduktion, ionische Wechselwirkungen, Säure-Base-Reaktionen, Ausbildung von Wasserstoffbrückenbindungen, Hydratisieren aber auch auf hydrolytische Vorgänge, und deren Kombinationen zurückzuführen. Diese potentiellen Interaktionen werden auch häufig gezielt dazu benutzt, eine Darreichungsform im Hinblick auf die Lösungsgeschwindigkeit, Löslichkeit, Bioverfügbarkeit, Stabilität, Minimierung von Nebenwirkungen optimal zu formulieren. Eine Salzbildung zwischen den sauren Gruppen der makromolekularen Carbonsäure und basischen Funktionen eines Arzneistoffmoleküls wird vielfach als Ursache für eine Interaktion diskutiert. Dabei können sich auch die Freisetzungseigenschaften ändern.

Im Zuge der Hydratisierung einer pharmazeutischen Darreichungsform im Verdauungstrakt können die Wirkstoffe auch in den Film der polymeren Carbonsäure migrieren, was zu Veränderungen der Freisetzungseigenschaften führen kann. Eindringende Feuchtigkeit setzt die Glastemperatur der Polymere in der Regel herab. Ähnliche Effekte kann die Migration des Arzneistoffs bewirken. Ein Absenken der Glastemperatur bewirkt in der Regel eine bessere Beweglichkeit der polymeren Strukturen und somit eine erhöhte Lösungsgeschwindigkeit. Andererseits kann einwandernder Wirkstoff durch Gerüstbildung zu einer höheren Festigkeit des Films beitragen, was eine verzögerte Auflösung bedeuten könnte. Arzneistoffe, die insbesondere in der An- und Auflösungsphase eines Polymers in den sauren Polymerfilm migrieren, können die Freisetzungseigenschaften positiv wie negativ beeinflussen. Negativ bedeutet in diesem Fall freisetzungsverzögernd. Hydrolytische Vorgänge am Polymer selbst bzw. beim Wirkstoff bzw. den pharmazeutischen Hilfsstoffen können ebenfalls die physikalisch-chemischen Eigenschaften eines Polymerfilms modifizieren. Polymere Carbonsäuren auf Basis der Cellulose sind diesbezüglich kritischer einzustufen als polymere Formen der Acrylsäure.

Umgekehrt gibt es polymere, basische Verbindungen, die beispielsweise im Magen protoniert werden und dann in kationischer Form gelöst vorliegen. Wie oben erwähnt sind auch die makromolekularen Verbindungen in der Regel in ihrer ionisierten Form zunehmend besser löslich.

Makromolekulare Carbonsäuren werden häufig als Überzugsmaterialien verwendet, da sie Schutzfunktionen haben können. Eine makromolekulare Carbonsäure kann als Überzugsmaterial u.a. einen säure-labilen Wirkstoff vor dessen Abbau im sauren Milieu des Magens schützen und/oder die Magenschleimhaut vor potentieller Schädigung durch den Arzneistoff schützen. Man spricht von magensaftresistenten Filmbildnern [4,5].

Typische Vertreter magensaftresistenter Filmbildner sind u.a.: Celluloseacetatphthalat, CAP, Aquateric®, von FMC; Celluloseacetattrimellitat, CAT; Hydroxypropylmethylcellulosephthalat, HPMCP, als HP-55, HP-55S und HP-50 von ShinEtsu; Methacrylsäure-Ethacrylat 1:1 Copolymere, MA-EA, Eudragit® L 30 D-55 und Eudragit® L 100-55 von Evonik; Methacrylsäure-Methylmethacrylat-1:1 und 1:2 Copolymere, MA-MMA, Eudragit® L 12.5 und Eudragit® S 12.5, Eudragit® L100 und Eudragit® S 100; Schellack, CertiSeal FC 300 und FC 300A von Mantrose-Haeuser; Carboxymethylethylcellulose CMEC®, von Freund Industrial Corporation; Polyvinylacetatphthalat, PVAP, Phthalavin® von Colorcon; Hydroxypropylmethylcelluloseacetatsuccinat, HPMCAS, Aqoat® AS-LF, Aqoat® AS-MF, Aqoat® AS-HF, Aqoat®AS-LG, Aqoat® AS-MG, Aqoat®AS-HG von ShinEtsu.

Alle genannten Filmbildner besitzen eine pH-abhängige Löslichkeit [4]. Die freien Carboxylgruppen an den Makromolekülen sorgen dafür, dass sich der Überzug bei höheren pH-Werten, die unter Deprotonierung eine Salzbildung ermöglichen, löst. Der Lösungs- bzw. Freigabe-pH-Wert der Filme ist von Material zu Material unterschiedlich [4]. Die Filmbildner werden typischerweise als organische und/oder wässrige Zubereitungen oder in Form von wässrigen Latex-Dispersionen verarbeitet. Zusammensetzungen und Prozessparameter sind in einschlägigen Lehrbüchern hinreichend beschrieben [4,5].

Besonders vorteilhaft im Sinne dieser Erfindung sind Überzugsverfahren mit polymeren Carbonsäuren, die in der Wirbelschicht ausgeführt werden, insbesondere Überzugsverfahren in der Wirbelschicht mit Führungszylindern wie sie in Wursteranlagen realisiert sind (Fa. Glatt GmbH).

Zur Vermeidung von Interaktionen von Kernbestandteilen, wie Wirkstoffen und Hilfsstoffen, mit magensaftresistenten Filmüberzugsmaterialien werden Zwischenschichten appliziert. Wirth und Bucher beschreiben doppelt beschichtete Granulate, wobei ein stark basischer Wirkstoff, der als Pelletkern formuliert ist, mit einer Zwischenschicht bestehend aus einer hydrophilen, elastischen inneren Beschichtung und nachfolgend mit einem magensaftresistenten, darmsaftlöslichen äußeren Überzug versehen ist [6]. In diesem Fall verhindert die eingezogene Trennschicht den direkten Kontakt des stark basischen Arzneistoffs mit dem Schutzüberzug, der sich bei direktem Kontakt an- bzw. auflösen könnte.

Eine Übersicht über Interaktionen von Wirkstoffen mit magensaftresistenten Filmbildnern und Maßnahmen, diese zu reduzieren bzw. diese zu unterbinden, findet sich in der Dissertation von A. Riedel, Universität Leipzig, Leipzig, 2004 [12]. Danach sind die potentiellen Wechselwirkungen auf sehr unterschiedliche Phänomene zurückzuführen.

Magensaftresistente Zubereitungen werden im Europäischen Arzneibuch [7] beschrieben, z.B. magensaftresistente Kapseln und magensaftresistente Tabletten. Die USP monographiert darüber hinaus magensaftresistent formulierte Spezialitäten [8]. Außerdem werden in den entsprechenden Monographien zugehörige *in vitro* Prüfungen, u.a. zur Bestimmung der Wirkstofffreisetzung aufgeführt [7]. Hier, siehe Abschnitt 2.9.3, werden mögliche Apparaturen, Ausführungen und explizite Auswertungskriterien angegeben. Beispielsweise darf gemäß Europäischem Arzneibuch, 6. Ausgabe, Grundwerk 2008, [7], auf Prüfstufe A1 mit saurer Prüfflüssigkeit, die die Passage durch den Magen simulieren soll, bei 6 Prüflingen kein einziger Wert mehr als 10 Prozent des in der Beschriftung des Arzneimittels angegebenen Gehaltes zeigen. Nachfolgend erfolgt eine Prüfstufe mit gepufferter Prüfflüssigkeit - die der Passage in den Darm entspricht - typischerweise Phosphatpuffer pH 6.8, wobei die Freisetzung möglichst vollständig erfolgen sollte, um auch eine vollständige Verfügbarkeit des Arzneistoffs im Verdauungstrakt zu gewährleisten.

Die Begrenzung der Wirkstofffreigabe auf maximal 10 % gilt hinlänglich als Nachweis und Beleg der Magensaftresistenz einer pharmazeutischen Zubereitung. Erst wenn die kumulative Freisetzung weniger als 10 % beträgt, spricht man von magensaftresistenten Zubereitungen. Demnach muss bei diesen Präparaten in der Regel so beschichtet werden, dass dieses Kriterium erreicht ist. Dies kann bei gleicher qualitativer und quantitativer Zusammensetzung eines magensaftresistenten Filmüberzugs mit zunehmendem Lackauftrag, d.h. zunehmender Schichtdicke, erzielt werden.

Darüber hinaus werden bei magensaftresistenten Formulierungen neben den eben beschrieben Prüfungen im Hinblick auf die Magensaftresistenz und die nachfolgende Prüfung in einem Phosphatpuffermedium auch Freisetzungsprüfungen mit den im Arzneibuch beschriebenen Apparaturen und Konditionen in einem Medium durchgeführt, das besonders nahe beim Auflösungs-pH-Wert des jeweiligen Polymers liegt. Auch hier sollte bei robusten Formulierungen möglichst eine komplette Freisetzung erreicht werden können. Bedingt durch die Klebrigkeit des Polymers und insbesondere bei möglicherweise bestehenden Wechselwirkungen mit Formulierungsbestandteilen inkl. Wirkstoff und dem sich auflösenden Polymer sind diese Prüfungen besonders anspruchsvoll, um eine vollständige Auflösung des Wirkstoffs selbst bei diesem Grenz-pH-Wert zu erreichen. Die vorliegende Erfindung zeigt, dass diese Aufgabenstellung außerordentlich gut gelöst wird.

Durch Nahrungszufuhr kann sich der pH-Wert des Magens so verschieben, dass das Polymer angelöst wird bzw. Feuchtigkeit in den Film penetrieren kann und somit zu einer Interaktion von Wirkstoff und Film beiträgt.

Die Wechselwirkungen von Arzneistoffen und Polymeren, insbesondere magensaftresistenten Polymeren, sind bekannt. Dies kann dazu führen, dass bedingt durch die Wechselwirkung zwischen kationischen Wirkstoffen und anionischen Polymeren bzw. auch anionischen Wirkstoffen und kationischen Polymeren, Wechselwirkungen eintreten, die bei magensaftresistenten Formulierungen sich dahingegen äußern, dass bedingt durch die ionischen Wechselwirkungen insbesondere in den Übergangsbereichen zur vollständigen Löslichkeit äußerst klebrige Massen entstehen, die ein vollständiges Auflösen des Wirkstoffs *in vitro* und möglicherweise auch *in vivo* verhindern bzw. unmöglich machen. In der Regel kann dieses Phänomen durch das Einziehen einer Zwischenschicht auf Basis von neutralen Filmbildnern, die keinen Einfluss auf die Wirkstofffreisetzung haben, und somit den Wirkstoff von dem magensaftresistenten Polymer trennt, unterbunden werden. Darüber hinaus können diesen verschiedenen Schichten Hilfsstoffe zugesetzt werden, die als Antiklebestoffe das Verkleben verhindern und somit eine verbesserte Auflösung ermöglichen. In der Regel kann durch diese Maßnahmen eine vollständige Freisetzung des Arzneistoffs aus der magensaftresistenten Formulierung erzielt werden. Es wurden jedoch auch schon Fälle beschrieben, wobei die eingezogenen Trennschichten durchaus einen Effekt auf die Freisetzung haben können. Dies ist im Einzelfall zu prüfen.

Einige pharmazeutische Wirkstoffe haben jedoch die Eigenschaft, dass selbst herkömmliche Trennschichten unterschiedlicher Dicke und qualitativer Zusammensetzung nicht ausreichen, um derartige Wechselwirkungen nahezu vollständig zu unterbinden. Dies ist insbesondere dann der Fall, wenn in quantitativer Hinsicht ausreichend Molekülstrukturen vorhanden sind und diese im Molekülverbund so lokalisiert sind, dass diese eine ungehinderte Interaktion eingehen können und schon bei geringster physikalischer Änderung des Polymerfilms ein Einwandern des Wirkstoffes in den Polymerfilm einsetzen kann, dem dann die Interaktion des Wirkstoffs mit dem Polymermaterial folgt. Solche Änderungen können z.B. schon bei geringem Feuchtigkeitseintritt in die Darreichungsform hervorgerufen werden. Für das Einwandern von Formulierungsbestandteilen in den Film oder auch von Wirkstoffen ist aber nicht notwendigerweise eine Änderung z.B. des Wassergehaltes notwendig. Manche Formulierungsbestandteile oder Wirkstoffe wandern bereits bei Lagerung.

Ein typischer Vertreter dieser besonders schwierig zu formulierenden Wirkstoffe ist Duloxetinhydrochlorid, ein kationischer Wirkstoff zur Behandlung von Depressionen. Duloxetinhydrochlorid muss magensaftresistent überzogen werden, da sich der Wirkstoff im sauren Milieu zu toxischem beta-Napthol umsetzt.

US 5 508 276, [9], beschreibt, dass vorzugsweise mit Hydroxypropylmethylcelluloseacetatsuccinat magensaftresistent beschichtete Duloxetinzubereitungen optimale Eigenschaften haben. Es konnte durch eigene Versuchsreihen bestätigt werden, dass sich mit anderen magensaftresistenten Filmbildnern (siehe oben) überzogene Duloxetinzubereitungen trotz klassischer Trennschicht wegen der anhaltenden Interaktion des kationischen Wirkstoffs nicht optimal herstellen lassen, da diese Präparate mit dem anionischen Polymer keine vollständige Auflösung in der nachfolgenden Pufferphase zeigen. Eine ausreichende Separierung von Wirkstoff durch eine klassische Trennschicht war daher nicht möglich, im Gegensatz zu Befunden von Kolatkar und Osinga [10, 11].

Der Erfindung liegt nun die Aufgabe zugrunde, pharmazeutische Zubereitungen in Pelletform bereitzustellen, welche vorzugsweise im Stand der Technik bekannte Schwierigkeiten beseitigen können und vorteilhafte Eigenschaften aufweisen. Dabei ist es insbesondere eine Aufgabe der Erfindung, magensaftresistent beschichtete Duloxetinpellets herzustellen, die nach Umpuffern auf der Prüfstufe mit gepufferter Prüflösung eine vollständige Freisetzung gewährleisten. Hierzu stellt die vorliegende Erfindung geeignete pharmazeutische Zubereitungen in Pelletform, magensaftresistente Duloxetinhydrochloridformulierungen auf Pelletbasis, pharmazeutische Zubereitungen in Pelletform sowie Darreichungsformen bereit.

Diese Zubereitungen sollen sich darüber hinaus auch im Grenzbereich der pH-Löslichkeit der zu verwendenden polymeren Carbonsäuren nahezu vollständig auflösen. Dabei sollen zur Prüfung die oben beschriebenen *in vitro*-Prüfmethoden eingesetzt werden.

Pelletprodukte mit potentiell interagierenden Wirkstoffen und Überzügen sind als besonders kritisch einzustufen, da hier im Vergleich zu einer herkömmlichen Tablettenformulierung, bedingt durch das Aufteilen der Wirkstoffdosis auf viele, z.T. mehrere hundert bis tausend Untereinheiten, den sogenannten multiple-units, infolge der erhöhten Oberfläche und des erhöhten Lackbedarfes, das Verhältnis von Wirkstoff zu Filmbildner im Hinblick auf eine mögliche Wechselwirkung besonders ungünstig und kritisch ist.

Zur Lösung der oben erläuterten Aufgaben der Erfindung wird in einem ersten Aspekt (Aspekt 1) eine pharmazeutische Zubereitung in Pelletform mit (i) einem Wirkstoff aufweisenden Kern, umfassend Duloxetin oder ein pharmazeutisch annehmbares Salz davon, insbesondere Duloxetinhydrochlorid, (ii) einer Trennschicht, umfassend einen Ester aus optional substituierter Cellulose mit einer oder mehreren organischen Säuren, (iii) einer magensaftresistenten Schicht, welche ein (Meth)acrylsäure-haltiges Polymer umfasst, wobei sich die Trennschicht (ii) zwischen dem Kern (i) und der magensaftresistenten Schicht (iii) befindet, und (iv) einer inneren Schutzschicht über dem Kern, die von der Trennschicht (ii) verschieden ist, sich zwischen dem Kern und der Trennschicht (ii) befindet und einen Zucker aufweist, bereitgestellt.

Die innere Schutzschicht entspricht einer ersten Trennschicht.

Zu dem ersten Aspekt der Erfindung gehören Zubereitungen, bei denen über dem wirkstoffhaltigen Kern eine innere Schutzschicht (erste Trennschicht) und über dieser inneren Schutzschicht eine davon verschiedene (zweite) Trennschicht vorliegt.

Erfindungsgemäß stellt eine pharmazeutische Zubereitung in Pelletform eine granuläre pharmazeutische Zubereitung dar.

Die Pellets der Zubereitung weisen typischerweise ein Kornspektrum zwischen 400 und 1200 µm, bevorzugt zwischen 500 und 1000 µm und insbesondere zwischen 600 und 900 µm auf. d50-Werte liegen vorzugsweise im Bereich von 500 bis 900 µm, vorzugsweise im Bereich von 600 bis 800 µm.

In einem bevorzugten zweiten Aspekt (Aspekt 2) ist der Ester in der Trennschicht ein optional substituiertes Celluloseacetat, ein optional substituiertes Cellulosesuccinat oder ein optional substituiertes

Celluloseacetatsuccinat ist, insbesondere Hydroxypropylmethylcelluloseacetatsuccinat.

In einem bevorzugten dritten Aspekt (Aspekt 3) ist in der pharmazeutischen Zubereitung gemäß Aspekt 1 oder 2 das (Meth)acrylsäure-haltige Polymer in der magensaftresistenten Schicht ein Methacrylsäure-Alkylacrylat, insbesondere Methacrylsäure-Ethylacrylat.

In einem bevorzugten vierten Aspekt (Aspekt 4) weist in der pharmazeutischen Zubereitung gemäß einem der Aspekte 1 bis 3 die innere Schutzschicht Rohrzucker, sowie optional ein wasserlösliches Polymer wie etwa Hydroxypropylmethylcellulose auf.

In einem bevorzugten fünften Aspekt (Aspekt 5) enthält die magensaftresistente Schicht in der pharmazeutischen Zubereitung gemäß einem der Aspekte 1 bis 4 kein Hydroxypropylmethylcelluloseacetatsuccinat.

In einem bevorzugten sechsten Aspekt (Aspekt 6) hat die magensaftresistente Schicht in der pharmazeutischen Zubereitung gemäß einem der Aspekte 1 bis 5 einen Anteil an der Zusammensetzung von = 20 Gew.-%, bevorzugt von = 16 Gew.-%, mehr bevorzugt von = 13 Gew.-% und insbesondere von = 10 Gew.-%.

In einem bevorzugten siebten Aspekt (Aspekt 7) ist die magensaftresistente Schicht in der pharmazeutischen Zubereitung gemäß einem der Aspekte 1 bis 6 so ausgebildet, dass sechs Proben einer solchen Zubereitung über zwei Stunden in 750 ml 0,1 N HCl bei 37 ± 0,5 °C im Drehkörbchen bei 100 U/min eine Freisetzung von Duloxetinhydrochlorid von = 10 % aufweisen.

Als Proben werden jeweils Kollektive von Pellets eingesetzt. Die Menge der Pellets in einer Probe wird dabei typischerweise so bemessen, dass sie einer Dosis von 20, 30, 40 oder 60 mg Wirkstoff, berechnet als Duloxetinbase, entspricht.

In einem bevorzugten achten Aspekt (Aspekt 8) ist die Trennschicht in der pharmazeutischen Zubereitung gemäß einem der Aspekte 1 bis 7 so ausgebildet, dass bei fehlender magensaftresistenter Schicht sechs Proben einer solchen Zubereitung über zwei Stunden in 750 ml 0,1 N HCl bei 37 ± 0,5 °C im Drehkörbchen bei 100 U/min eine Freisetzung von Duloxetinhydrochlorid von > 10 % bei mindestens einer der sechs Zubereitungen aufweisen.

Liegt also lediglich die Trennschicht, nicht aber die darauf vorgesehene magensaftresistente Schicht vor, so ist keine Magensaftresistenz gegeben. In Bezug auf die Probe gilt das vorstehend Gesagte.

In einem bevorzugten neunten Aspekt (Aspekt 9) weist eine Probe der pharmazeutischen Zubereitung gemäß einem der Aspekte 1 bis 8 in 1000 ml Acetatpufferlösung mit einem pH-Wert von 5,5 bei 37 ± 0,5 °C im Drehkörbchen bei 100 U/min eine Freisetzung von = 90 % des Wirkstoffes nach einem Zeitraum von = 7 Stunden, bevorzugt = 5 Stunden, mehr bevorzugt = 4 Stunden und insbesondere = 3 Stunden auf.

In Bezug auf die Probe gilt das vorstehend Gesagte.

In einem bevorzugten zehnten Aspekt (Aspekt 10) besteht bei der pharmazeutischen Zubereitung gemäß einem der Aspekte 1 bis 9 der Wirkstoff aufweisende Kern aus einem wirkstofffreien inerten inneren Kern sowie einer Wirkstoffschicht über dem inneren Kern, wobei die Wirkstoffschicht Duloxetin oder ein pharmazeutisch annehmbares Salz davon, insbesondere Duloxetinhydrochlorid, umfasst.

In einem bevorzugten elften Aspekt (Aspekt 11) besteht bei der pharmazeutischen Zubereitung gemäß einem der Aspekte 1 bis 9 der Wirkstoff aufweisende Kern aus einem Matrixmaterial, in welches Duloxetin oder ein pharmazeutisch annehmbares Salz davon, insbesondere Duloxetinhydrochlorid, eingebettet ist.

Ferner stellt die vorliegenden Erfindung in einem zwölften Aspekt (Aspekt 12) eine magensaftresistente Duloxetinhydrochloridformulierung in Pelletform bereit, welche einen Pelletaufbau aus (i) einem inerten Kern, (ii) einer Wirkstoffschicht über dem Kern, welche Duloxetinhydrochlorid, Hydroxypropylmethylcellulose und Talkum aufweist, (iii) einer inneren Schutzschicht (ersten Trennschicht) über der Wirkstoffschicht, welche Hydroxypropylmethylcellulose, Rohrzucker und Talkum aufweist, (iv) einer von der inneren Schutzschicht (ersten Trennschicht) verschiedenen (zweiten) Trennschicht über der inneren Schutzschicht, welche Hydroxypropylmethylcelluloseacetatsuccinat, Talkum und Triethylcitrat aufweist, und (v) einer von der inneren Schutzschicht und der (zweiten) Trennschicht verschiedenen, magensaftresistenten Schicht, welche Methacrylsäure-Ethylacrylat-Copolymer, Triethylcitrat, Talkum und Natriumhydroxid aufweist, hat. Gemäß einer bevorzugten Ausführungsform bildet die magensaftresistente Schicht die äußere Schicht des Pellets.

Ferner stellt die vorliegenden Erfindung in einem dreizehnten Aspekt (Aspekt 13) eine magensaftresistente Duloxetinhydrochloridformulierung in Pelletform bereit, welche einen Pelletaufbau aus (i) einem Kern bestehend aus Duloxetinhydrochlorid in einer Matrix aus mikrokristalliner Cellulose, quervernetztem Polyvinylpyrrolidon und Mannitol, (ii) einer inneren Schutzschicht (ersten Trennschicht) über der Wirkstoffschicht, welche Hydroxypropylmethylcellulose, Rohrzucker und Talkum aufweist, (iii) einer von der inneren Schutzschicht verschiedenen (zweiten) Trennschicht über der inneren Schutzschicht, welche Hydroxypropylmethylcelluloseacetatsuccinat, Talkum und Triethylcitrat aufweist, und (iv) einer von der inneren Schutzschicht und der (zweiten) Trennschicht verschiedenen, magensaftresistenten Schicht, welche Methacrylsäure-Ethylacrylat-Copolymer, Triethylcitrat, Talkum und Natriumhydroxid aufweist, hat. Gemäß einer bevorzugten Ausführungsform bildet die magensaftresistente Schicht die äußere Schicht des Pellets.

Die Erfindung stellt des weiteren eine Darreichungsform bereit, welche eine granuläre pharmazeutische Zubereitung gemäß einem der Aspekte 1 bis 11 oder eine magensaftresistente Duloxetinhydrochloridformulierung gemäß Aspekt 12 oder 13 enthält, wobei die Darreichungsform eine Tablette oder eine Kapsel ist, bevorzugt eine Kapsel und mehr bevorzugt eine Hartkapsel.

In einer bevorzugten Ausführungsform der Erfindung schließt die magensaftresistente Schicht die erfindungsgemäßen pharmazeutischen Zubereitungen in Pelletform ab. Es schließt sich keine Außenphase und keine weitere Schicht an.

In einer weiteren bevorzugten Ausführungsform der Erfindung sind die Zubereitungen bzw. Formulierungen im Sinne dieser Erfindung über der magensaftresistenten Schicht zusätzlich mit einer Außenphase und/oder mit einer weiteren Schicht überzogen.

Erfindungsgemäß werden ferner zu überziehende Darreichungsformen, z.B. Duloxetinhydrochlorid-haltige pharmazeutische Zubereitungen wie Granulate, Pellets und Tabletten, hergestellt. Dabei wird der Wirkstoff, vorzugsweise in vollständig gelöster Form, auf Neutralpellets wie Sugar Spheres bestehend aus Stärke und Rohrzucker, mikrokristalline Cellulosepellets oder Mannitolpellets in der Wirbelschicht aufgelayert. Bevorzugt sind hier nahezu vollständig wasserlösliche Starterkerne wie z.B. Sugar Spheres oder Mannitolpellets. Diese Wirkstoffpellets werden zunächst (und optional) mit einer Hydroxypropylmethylcellulose oder einem anderen vergleichbaren neutralen Filmbildner enthaltenden ersten Zwischenschicht (innere Schutzschicht oder erste Trennschicht) überzogen und nachfolgend mit einer, die Wechselwirkung verhindernden oder unterbindenden Zwischenschicht, mit Hydroxypropylmethylcelluloseacetatsuccinat als Filmbildner so beschichtet, dass die Wirkstofffreisetzung dieser mehrfach beschichteten Zubereitungen in saurer Prüfflüssigkeit > 10 % Wirkstofffreisetzung erreicht.

Nachfolgend kann das mit der Trennschicht/den Trennschichten überzogene Pellet mit einem klassischen magensaftresistenten Film überzogen werden, um Magensaftresistenz im Sinne der Arzneibücher zu erreichen, wobei bei sequentieller Prüfung auf Prüfstufe A1 gemäß Europäischem Arzneibuch 2008 [7] bei 6 geprüften Einheiten kein einziger Wert mehr als 10 % beträgt. In besonders geeigneten Ausführungsformen können die einzelnen Carboxylgruppen der die Wechselwirkung verhindernden HPMCAS-haltigen Trennschicht und die der magensaftresistenten Schicht während der Applikation auch alkalisiert bzw. teilneutralisiert sein, was im Falle von HPMCAS z.B. durch Ammoniaklösung, bei dem magensaftresistenzbildenden Methacrylsäure-Methacrylat-Copolymer z.B. durch Natronlauge erzielt wird. Ammoniaklösung verflüchtigt sich während der nachfolgenden Trocknung. Neben den bereits beschriebenen Methacrylsäure-Methacyrylat- bzw. Methacrylsäure-Ethylacrylat-Copolymeren können auch andere Filmbildner für magensaftresistente Überzüge mit Ausnahme von Hydroxypropylmethylcellululoseacetatsuccinat eingesetzt werden.

Dabei verhindert insbesondere der Trennschichtaufbau eine auch während der Prüfung im sauren Milieu stattfindende Migration von Feuchtigkeit in den Duloxetinhydrochlorid-haltigen Pelletkern, der ein Anlösen des Wirkstoffs und ein Einwandern in die finale Magensaftresistenz-liefernde äußere Schicht, die kein Hydroxypropylmethylcelluloseacetatsuccinat enthält, unterbindet. Konsequenterweise ergibt sich, dass das Polymer ungehindert in Lösung gehen kann, bevor es mit dem Wirkstoff unter Ausbildung eines sich nur zögernd auflösenden Produktes interagiert.

Diese beschichteten Zubereitungen sind weiterhin dadurch charakterisiert, dass sie im Prüfmedium im pH-Grenzbereich zur Löslichkeit der polymeren Carbonsäure auch eine vollständige Freisetzung zeigen und kein abflachendes Auflösungsprofil ergeben, das eine unvollständige Freisetzung des Wirkstoffs aus der Darreichungsform im Sinne dieser Erfindung anzeigt.

Die Herstellung der Zubereitungen im Sinne dieser Erfindung kann wie nachstehend beschrieben erfolgen:
Stufe A: Beschichtung von Neutralpellets mit Duloxetinhydrochlorid

Dazu werden aus Neutralpellets, z.B. bestehend aus Stärke und Rohrzucker, in der Wirbelschicht (GPCG 1, Firma Glatt GmbH, Binzen) mit einer Layeringdispersion besprüht. Zur Herstellung der Layeringdispersion wird absoluter Ethanol in einem Edelstahlbehälter vorgelegt und nachfolgend mit Reinwasser 1:1 verdünnt. In der Mischung wird der Wirkstoff Duloxetinhydrochlorid und dann das Bindemittel Hydroxypropylmethylcellulose (Pharmacoat 606, Shin-Etsu) gelöst. Zuletzt wird Talkum als Trennmittel in die Lösung eingerührt. Der Ansatz wird kontinuierlich weitergerührt. Die Wirbelschichtanlage mit eingebautem 6" Wurstereinsatz wird mit den Neutralpellets bestückt und anschließend mit der Layeringdispersion bei einer Zulufttemperatur von etwa 50 ± 5 °C und 2 bar Sprühdruck besprüht. Die Luftmenge während der Sprühphase beträgt etwa 120 m³/h. Die Produkttemperatur beträgt etwa 40 °C. Die Sprührate beträgt vorzugsweise etwa 5 - 10 g/Minute. Nachfolgend werden die beschichteten Pellets für etwa 20 Minuten bei 45 °C Produkttemperatur getrocknet. Der LOD Wert beträgt < 1 % (Halogentrockner Fa. Mettler, 105 °C, 1 mg/30s). Nach dem Beschichten mit Wirkstoff werden Pellets mit einem Kornspektrum zwischen 500 - 800 µm erhalten (d50 ∼ 675 µm). Die quantitative Zusammensetzung der zuvor beschriebenen Formulierung ist nachfolgend beschrieben:

| Material | Produktbezeichnung, Hersteller | Masse (g) |
|---|---|---|
| Neutralpellets 500 - 600 µm | Pharm-a-spheres, Werner | 500,00 |
| Duloxetinhydrochlorid | | 300,00 |
| Hydroxypropylmethylcellulose | Pharmacoat 606, Shin-Etsu | 96,00 |
| Talkum | | 4,00 |
| Reinwasser (verdampft während Prozess) | | 1133,00 |
| Ethanol, absolut (verdampft während Prozess) | | 1133,00 |
| Ausbeute | | 900,00 |

An dieser Stelle soll ausdrücklich darauf hingewiesen werden, dass Beschichtung von Neutralpellets mit Duloxetinhydrochlorid auch wässrig gearbeitet werden kann. Dazu werden aus Neutralpellets, z.B. bestehend aus Stärke und Rohzucker, in der Wirbelschicht (GPCG 1, Firma Glatt GmbH, Binzen) mit einer Layeringdispersion besprüht. Zur Herstellung der Layeringdispersion wird der Wirkstoff Duloxetinhydrochlorid (Partikelgröße < 50 µm, mittlere Partikelgröße: 20 µm) und in Reinwasser dispergiert und dann das Bindemittel Hydroxypropylmethylcellulose (Pharmacoat 606, Shin-Etsu) gelöst. Zuletzt wird Talkum als Trennmittel in die Lösung eingerührt. Der Ansatz wird kontinuierlich weitergerührt. Die Wirbelschichtanlage mit eingebautem 6" Wurstereinsatz wird mit den Neutralpellets bestückt und anschliessend mit der Layeringdispersion bei einer Zulufttemperatur von etwa 60 ± 5 °C und 2 bar Sprühdruck besprüht. Die Luftmenge während der Sprühphase beträgt etwa 120 m³/h. Die Produkttemperatur beträgt etwa 40 °C. Die Sprührate beträgt vorzugsweise etwa 5 - 10 g/Minute. Nachfolgend werden die beschichteten Pellets für etwa 30 Minuten bei 45 °C Produkttemperatur getrocknet. Der LOD Wert beträgt < 1 % (Halogentrockner Fa. Mettler, 105 °C, 1 mg/30s). Nach dem Beschichten mit Wirkstoff werden Pellets mit einem Kornspektrum zwischen 500 - 800 µm erhalten (d50 ∼ 675 µm). Die quantitative Zusammensetzung der zuvor beschriebenen Formulierung ist nachfolgend beschrieben:

| Material | Produktbezeichnung, Hersteller | Masse (g) |
|---|---|---|
| Neutralpellets 500 - 600 µm | Pharm-a-spheres, Werner | 500.00 |
| Duloxetinhydrochlorid (Partikelgröße < 50 µm, mittlere Partikelgröße: 20 µm) | | 300.00 |
| Hydroxypropylmethylcellulose | Pharmacoat 606, Shin-Etsu | 96.00 |
| Talkum | | 4.00 |
| Reinwasser (verdampft während Prozess) | | 2267.00 |
| Ausbeute | | 900.00 |

Es wird ferner darauf hingewiesen, dass Wirkstoffpellets im Sinne dieser Erfindung auch durch geeignete Granulationsverfahren so aufgebaut werden können, dass der Wirkstoff, insbesondere Duloxetinhydrochlorid, zusammen mit geeigneten Hilfsstoffen vorbefeuchtet wird (z.B. im Schnellmischer VG 10) und die vorbefeuchtete Masse mittels Extrusion-Sphäronisation oder auch in der Rotorwirbelschicht zu Pellets geeigneter Größe verarbeitet wird. Der Ausrundung schließt sich typischerweise eine Trocknung in der Wirbelschicht bzw. im Hordentrockenschrank an.

Die Zusammensetzung einer solchen Kernformulierung, die den Wirkstoff Duloxetinhydrochlorid enthält, ist in der nachstehenden Tabelle wiedergegeben. Danach besteht ein solcher Kern neben dem Wirkstoff vorzugsweise aus mikrokristalliner Cellulose (Avicel PH 105, FA. FMC), Mannitol (Pearlitol 160, Fa. Roquette) und quervernetztem Polyvinylpyrrolidon (Kollidon CL, Fa. BASF).

| **Kernformulierung** | **Masse (g)** |
|---|---|
| Duloxetinhydrochlorid | 350,00 |
| Mikrokristalline Cellulose (Avicel PH 105 von FMC) | 200,00 |
| Quervernetztes Polyvinylpyrrolidon (Kollidon CL-M von BASF) | 100,00 |
| Mannitol (Pearlitol 160 C von Roquette) | 350,00 |
| Ausbeute | 1000,00 |

Die in der Tabelle angegebene Pulvermischung wird im Schnellmischer VG 10 (Fa. Glatt, Binzen) für 2 Minuten gemischt und über eine 1.0 mm Zweistoffdüse mit 200 g gereinigtem Wasser befeuchtet. Die Prozessparameter sind nachstehend beschrieben.

| **Vorbefeuchtung (Glatt VG 10)** | |
|---|---|
| Impellet- /Zerhackerdrehzahl | 100/2000 UpM |
| Sprühdruck | 0,5 bar |
| Sprührate | 20 ± 5 g / min |
| Wassermenge | 200,0 g |
| Trocknungsverlust | ∼ 15 % |

Nach der Befeuchtung hat die Masse einen Trocknungsverlust, der mit dem Mettler Halogentrockner bestimmt wird, von etwa ∼ 15 %.

Die so vorbefeuchtete Masse wird nachfolgend unter Besprühen mit gereinigtem Wasser in der Rotorwirbelschicht, z.B. im Schaufelrotor, CPS 3, Fa. Glatt, Binzen, zu Pellets ausgerundet und in der Wirbelschicht getrocknet (GPCG 1, Fa. Glatt, Binzen). Der LOD Wert beträgt < 1 % (Halogentrockner Fa. Mettler, 105 °C, 1 mg/30s). Nach Trocknung werden Pellets mit einem Kornspektrum zwischen 400-1000 µm erhalten (d50 ∼ 700 µm).

| **Verrundung (Glatt CPS 3)** | |
|---|---|
| Rotorplatte, Winkel | 30 ° |
| Anzahl Schaufeln im Prozessraum | 4 flache Schaufeln |
| Zulufttemperatur | 30 ± 2 ° C |
| Zuluftvolumen | 50 ± 10 m³/h |
| Rotorgeschwindigkeit während der Sprühphase | 500 ± 100 UpM |
| Sprührate | 20 ± 5 g / min |
| Wassermenge | 450,0 g |
| Rotorgeschwindigkeit während finaler Ausrundung (2 Minuten nach Sprühphase) | 500 ± 100 UpM |
| Trocknungsverlust nach Ausrundung | ∼ 35 % |

| **Trocknung (Glatt GPCG 1)** | |
|---|---|
| Zulufttemperatur | 60 ± 5 ° C |
| Zuluftvolumen | 175 ± 25 m³/h |
| Produkttemperatur bei Trocknungsende | 50 ± 1 ° C |
| Trocknungsverlust nach der Trocknung | 0,61 % |

Hinsichtlich der Prozessführung ist dieses alternative Verfahren zur Herstellung von Duloxetinhydrochlorid-haltigen Kernen gegenüber dem Layering von Duloxetinhydrochlorid auf inerte Startermaterialien als komplexer einzustufen. Darüber hinaus ist durch den Einsatz von sehr definierten Startermaterialien u.a. im Hinblick auf die Partikelgröße und des Partikelgrößenspektrums der Startermaterialien, eine gute, reproduzierbare und scale-up fähige Prozessführung möglich. Aus prozesstechnischen und ökonomischen Gesichtspunkten ist demzufolge die Herstellung von Duloxetinhydrochloridpellets mittels Layering besonders vorteilhaft, zumal der Wirkstoff im oben angegeben Fall gelöst verarbeitet werden kann und Schwankungen der Produkteigenschaften (z.B. Dissolution), verursacht durch eine variable Teilchengrößenverteilung des Wirkstoffs deutlich reduziert, wenn nicht sogar ausgeschlossen sind, da der gesamte Wirkstoff zuvor aufgelöst werden kann.

### Stufe B: Zwischenschicht 1

700 g der wie oben beschriebenen Duloxetinhydrochloridpellets werden nachfolgend in der Wirbelschicht (GPCG I, Fa. Glatt, Binzen, D) mit einer wässrigen Dispersion bestehend aus Hydroxypropylmethylcellulose (Pharmacoat 603) und Rohrzucker als Bindemittel und Filmbildner, sowie Talkum als Trennmittel beschichtet. Dazu wird Reinwasser in einem geeigneten Edelstahlbehälter vorgelegt. Nachfolgend werden die Hydroxypropylmethylcellulose und Zucker unter Rühren gelöst. Abschließend wird Talkum in der Lösung dispergiert. Die Wirbelschichtanlage mit eingebautem 6" Wurstereinsatz wird mit den Duloxetinhydrochloridpellets bestückt und anschließend mit der Dispersion bei einer Zulufttemperatur von etwa 55 ± 5 °C und 2 bar Sprühdruck besprüht. Die Luftmenge während der Sprühphase beträgt etwa 120 ± 20 m³/h. Die Produkttemperatur beträgt etwa 45 °C. Die Sprührate beträgt vorzugsweise etwa 5 - 10 g/Minute. Nachfolgend werden die beschichteten Pellets für etwa 20 Minuten bei 45 °C Produkttemperatur getrocknet. Der LOD Wert der getrockneten Pellets beträgt < 1 % (Halogentrockner Fa. Mettler, 105 °C, 1 mg/30s). Nach dem Beschichten mit Wirkstoff werden Pellets mit einem Kornspektrum zwischen 630 - 800 µm erhalten (d50 ∼ 680 µm). Die quantitative Zusammensetzung der Formulierung ist nachfolgend beschrieben:

| Material | Produktbezeichnung, Hersteller | (g) |
|---|---|---|
| Duloxetinhydrochloridpellets | - | 700,00 |
| Hydroxypropylmethylcellulose | Pharmacoat 603, Shin-Etsu | 15,00 |
| Rohrzucker | | 30,00 |
| Talkum | | 60,00 |
| Reinwasser^{(verdampft während Prozess)} | | 420,00 |
| | Ausbeute | 805.00 |

### Stufe C: Zwischenschicht 2

700 g der wie oben mit Hydroxypropylmethylcellulose und Rohrzucker befilmten Duloxetinhydrochloridpellets werden in der Wirbelschicht (GPCG I, Fa. Glatt, Binzen, D) mit einer wässrigen Dispersion bestehend aus dem Filmbildner Hydroxypropylmethylcelluloseacetatsuccinat (Aqoat AS-LG, Shin-Etsu), dem Weichmacher Triethylcitrat, dem Trennmittel Talkum und 25%iger Ammoniaklösung zur Alkalisierung des Ansatzes und Reinwasser beschichtet.

Dazu wird zunächst das Reinwasser in einem geeigneten Edelstahlbehälter vorgelegt. Nachfolgend wird Triethylcitrat mit dem Ultraturrax eingearbeitet und die Ammoniaklösung zugegeben. Abschließend wird das Aqoat in der wässrigen Zubereitung gelöst und Talkum unter Rühren eingearbeitet. Der Ansatz wird kontinuierlich weitergerührt.

Die Wirbelschichtanlage mit eingebautem 6" Wurstereinsatz wird mit den zuvor befilmten Pellets bestückt und anschließend mit der Dispersion bei einer Zulufttemperatur von etwa 50 ± 5 °C und 2 bar Sprühdruck besprüht. Die Luftmenge während der Sprühphase beträgt etwa 130 ± 20 m³/h. Die Produkttemperatur beträgt etwa 40 °C. Die Sprührate beträgt vorzugsweise etwa 5 - 15 g/Minute. Nachfolgend werden die beschichteten Pellets für etwa 30 Minuten bei 45 °C Produkttemperatur getrocknet. Der LOD Wert dieser Pellets beträgt < 1 % (Halogentrockner Fa. Mettler, 105 °C, 1 mg/30s). Nach dem Beschichten mit Wirkstoff werden Pellets mit einem Kornspektrum zwischen 630 - 900 µm erhalten (d50 ∼ 750 µm). Die quantitative Zusammensetzung der Formulierung ist nachfolgend beschrieben:

| Material | Produktbezeichnung, Hersteller | (g) |
|---|---|---|
| Beschichtete Duloxetinhydrochloridpellets | - | 700,00 |
| Hydroxypropylmethylcelluloseacetatsuccinat | Aqoat AS-LG, Shin-Etsu | 153,00 |
| Triethylcitrat | | 15,00 |
| Talkum | | 42,00 |
| Ammoniumhydroxidlösung 25 %^{(verdampft während Prozess)} | | 16,00 |
| Reinwasser^{(verdampft während Prozess)} | | 1874,00 |
| | Ausbeute | 910,00 |

### Stufe D: Magensaftresistente Schicht

800 g der schon zweifach befilmten Duloxetinhydrochloridpellets werden nachfolgend in der Wirbelschicht (GPCG I, Fa. Glatt, Binzen, D) mit einer wässrigen Dispersion bestehend aus dem Filmbildner Methacrylsäure-Ethylacrylat Copolymer (Eudragit® L 30 D, Evonik), dem Weichmacher Triethylcitrat, dem Trennmittel Talkum und Natriumhydroxid zur teilweisen Neutralisation der polymeren Carbonsäuren und Reinwasser beschichtet. Die so magensaftresistent beschichteten Pellets werden abschließend mit Talkum gemischt, um ein Verkleben der Pellets während der Lagerung zu verhindern.

Dazu wird zunächst Reinwasser in einem geeigneten Edelstahlbehälter vorgelegt. Nachfolgend werden Triethylcitrat und Talkum mit dem Ultraturrax unter Rühren eingearbeitet und die Polyacrylsäuredispersion zugegeben. Zuletzt wird eine wässrige Lösung von Natriumhydroxid in Reinwasser zur Dispersion gegeben und gerührt.

Die Wirbelschichtanlage mit eingebautem 6" Wurstereinsatz wird mit den zuvor befilmten Pellets bestückt und anschließend mit der Dispersion bei einer Zulufttemperatur von etwa 40 ± 5 °C und 2 bar Sprühdruck besprüht. Die Luftmenge während der Sprühphase beträgt etwa 120 ± 20 m³/h. Die Produkttemperatur beträgt etwa 31 °C. Die Sprührate beträgt vorzugsweise etwa 5 - 10 g/Minute. Nachfolgend werden die beschichteten Pellets für etwa 20 Minuten bei 40 °C Produkttemperatur getrocknet. Der LOD Wert der magensaftresistenten Pellets beträgt = 1 % (Halogentrockner Fa. Mettler, 105 °C, 1 mg/30s). Nach dem Beschichten mit Wirkstoff werden Pellets mit einem Kornspektrum zwischen 710 - 900 µm erhalten (d50 ∼ 760 µm). Die Gutfraktion wird im Kontainermischer (CM 10, Glatt GmbH, Binzen) für 5 Minuten mit Talkum gemischt. Die quantitative Zusammensetzung der Formulierung ist nachfolgend beschrieben:

| Material | Produktbezeichnung, Hersteller | (g) |
|---|---|---|
| Beschichtete Duloxetinhydrochloridpellets | - | 800,00 |
| Methacrylsäure-Ethylacrylat Copolymer, wässrige Dispersion ^{(70 % verdampft während Prozess)} | Eudragit® L 30 D, Evonik | 222,00 |
| Triethylcitrat | | 7,00 |
| Talkum | | 7,00 |
| Reinwasser^{(verdampft während Prozess)} | | 142,00 |
| Natriumhydroxid | | 0,88 |
| Reinwasser^{(verdampft wahrend Prozess)} | | 21,12 |
| Talkum | | 4,41 |
| | Ausbeute | 885,89 |

In einer bevorzugten Ausführungsform der Erfindung ist die Herstellung der erfindungsgemäßen Zubereitung mit der Ausbildung der magensaftresistenten Schicht abgeschlossen.

In einer weiteren bevorzugten Ausführungsform der Erfindung können die Zubereitungen im Sinne dieser Erfindung nach Beschichtung mit der magensaftresistenten Schicht zusätzlich mit einer Außenphase und/oder mit einer weiteren Schicht überzogen werden.

Der optionale finale Schichtauftrag fungiert als Schutz- und Trennschicht und wird ebenfalls mit Hilfe der zuvor beschriebenen Wirbelschichtapparaturen, vorzugsweise unter Einsatz wasserlöslicher Polymere als Filmbildner, aus wässriger Dispersion auf die Pellets aufgesprüht. Dabei kann in der Regel auf die weiter oben beschriebene Außenphase verzichtet werden. Neben den Filmbildnern (Hydroxypropylmethylcellulose, Hydroxypropylcellulose, Polyvinylpyrrolidon, etc.) werden hier übliche Trennmittel (Talkum, kolloidale Kieselsäure, etc.), Opakisierungsmittel (Titandioxid, etc.), Weichmacher (Macrogol, Triethylcitrat etc.) und Farbstoffe (Eisenoxide etc.) zur Herstellung einer geeigneten Formulierung für die Schutzschicht aufgetragen. Eine solche zusätzliche Trennschicht hat in der Regel keinen Einfluss auf die Freisetzungseigenschaften der Darreichungsform.

Die hergestellten Duloxetinzubereitungen können in Hartkapseln unterschiedlicher Dosierung verkapselt werden. Darüber hinaus können vergleichbar aufgebaute und beschichtete Zubereitungen auch zu Tabletten verpresst werden. Vorzugsweise werden die Pellets jedoch verkapselt.

Die quantitative Zusammensetzung der festen Bestandteile der Rezeptur mit magensaftresistent-beschichteten Duloxetinhydrochloridpellets mit einer Dosierung kalkuliert für 60 mg Duloxetinbase ist in nachstehender Tabelle wiedergegeben:

| **Material** | **Menge für 60 mg Duloxetin Dosis** |
|---|---|
| **(Name)** | **(mg)** |
| **Starterpellets (33.6%)** | |
| Pharm-a-spheres, 500-600 µm (Neutral pellets) | 112,33 |

| **Stufe A: Wirkstoffschicht (26.9 %)** | |
|---|---|
| Duloxetinhydrochlorid | 67,40 |
| Pharmacoat 606 (HPMC) | 21,57 |
| Talkum | 0,90 |
| Reinwasser | - |
| Ethanol, absolut | - |

| **Stufe B: Erste Trennschicht (9.1 %)** | |
|---|---|
| Pharmacoat 603 (HPMC) | 4,34 |
| Rohrzucker | 8,67 |
| Talkum | 17,32 |

| **Stufe C: Zweite Trennschicht (20.9 %)** | |
|---|---|
| Aqoat AS-LG (HPMCAS) | 50,71 |
| Talkum | 13,95 |
| Triethylcitrat | 5,10 |
| Reinwasser | - |
| Ammoniaklösung, 25 %ig | - |

| **Stufe D: Magensaftresistente Schicht (9.1 %)** | |
|---|---|
| Methacrylsäure-Ethylacrylat Copolymer | 25,21 |
| Triethylcitrat | 2,51 |
| Talkum | 2,51 |
| Natriumhydroxid | 0,34 |
| Reinwasser | - |

| **Außenphase (0.5 %)** | |
|---|---|
| Talkum | 1,66 |
| Total | 334,52 |

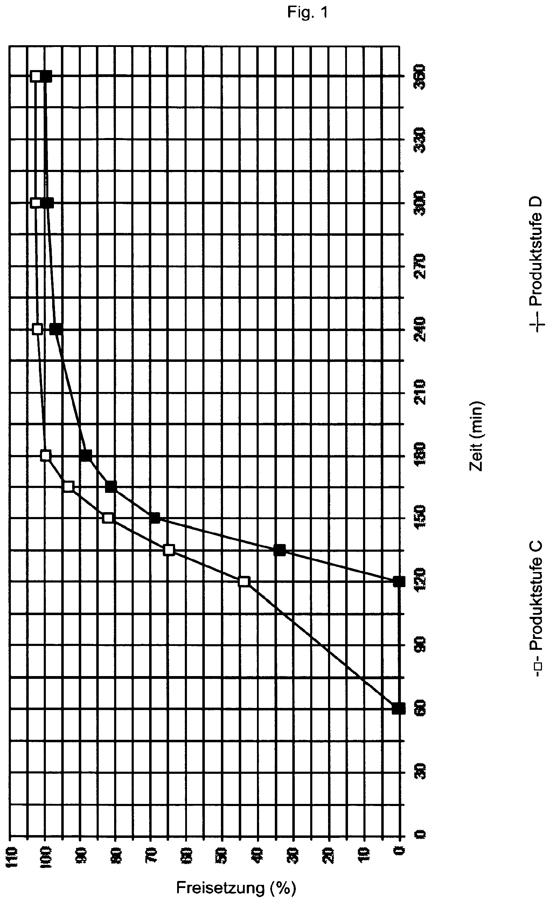

Danach weist die magensaftresistenzbildende Schicht einen massebezogenen Formulierungsanteil von nur etwa 9.1 % der Gesamtmasse der Pelletzubereitung zur Abfüllung in Hartkapseln auf. Beispielhafte Freisetzungsprofile der magensaftresistenten Formulierung für die Produktstufen C und D sind nachstehend aufgeführt:
Freisetzung der Zubereitungen der Produktstufen C und D:
Auf dieser Produktstufe ist das Präparat nicht magensaftresistent; die kumulative Freisetzung liegt weit über 10 % der enthaltenen Dosis. Durch die nachfolgende Beschichtung mit dem magensaftresistenten Filmbildner ergibt sich die geforderte Magensaftresistenz mit einer nachfolgend vollständigen Freisetzung des gesamten Wirkstoffs in der sich anschließenden Pufferphase. Die Freisetzung von den erfindungsgemäßen Duloxetinhydrochloridprodukten über 2 Stunden in 750 ml 0,1 n HCl mit nachfolgender Umpufferung durch Zugabe von 250 ml Phosphatpufferkonzentrat auf insgesamt 1000 ml mit pH 6,8 bei 37 °C im Drehkörbchen mit 100 U/min ist in Figur 1 gezeigt.

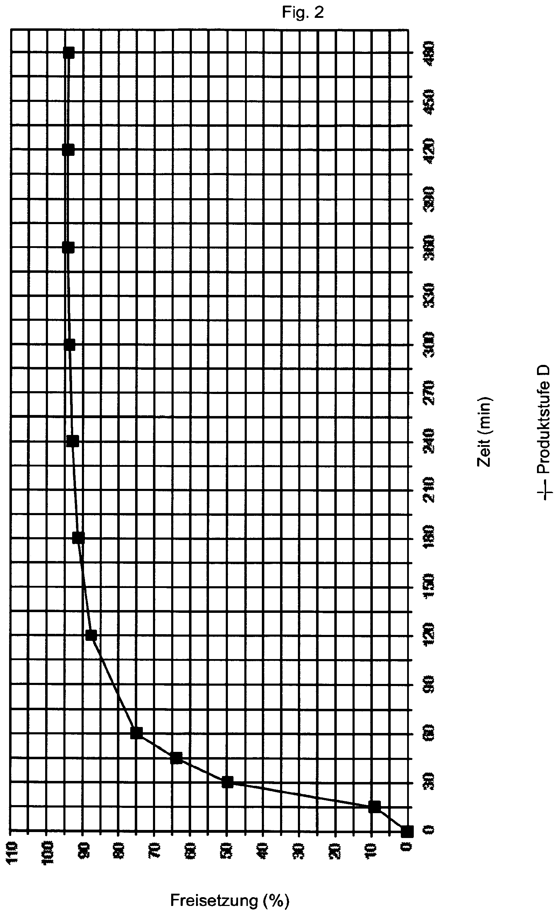

Führt man die Freisetzungsprüfung der Duloxetinhydrochloridzubereitungen bei einem pH-Wert im Grenzbereich der Löslichkeit der polymeren Carbonsäuren durch, ergeben sich ebenfalls Profile, die eine vollständige Duloxetinhydrochloridfreisetzung zeigen. Die Freisetzung von den erfindungsgemäßen Duloxetinhydrochloridprodukten über 7 Stunden in 1000 ml Acetatpufferlösung gemäß Europäischem Arzneibuch, 6. Ausgabe, bei pH 5,5 und 37 °C im Drehkörbchen bei 100 U/min ist in Figur 2 gezeigt.
[1] Schunack, W., Arzneistoffe, 1981
[2] Aktories, K., Repetitorium Allgemeine und Spezielle Pharmakologie und Toxikologie, 2006
[3] Schmidt, R., Physiologie Kompakt, 1999
[4] Ritschel, W., Die Tablette, 2002
[5] Bauer, K., Überzogene Arzneiformen, 1988
[6] EP 0421921B1, Wirth, D.
[7] Europäisches Arzneibuch, 6. Ausgabe, 2008
[8] USP 31/NF 26, 2008
[9] US Patent 5508276, Anderson, N.
[10] WO2007/139886A2, Kolatkar, G.
[11] WO2009/010238A2, Osinga, N.
[12] Riedel, A., Dissertation Universität Leipzig, 2004

## Patentansprüche

1. Pharmazeutische Zubereitung in Pelletform, umfassend
(i) einen Wirkstoff aufweisenden Kern, umfassend Duloxetin oder ein pharmazeutisch annehmbares Salz davon, insbesondere Duloxetinhydrochlorid,
(ii) eine Trennschicht, umfassend einen Ester aus optional substituierter Cellulose mit einer oder mehreren organischen Säuren,
(iii) eine von der Trennschicht verschiedene magensaftresistente Schicht, welche ein (Meth)acrylsäure-haltiges Polymer umfasst, wobei sich die Trennschicht (ii) zwischen dem Kern (i) und der magensaftresistenten Schicht (iii) befindet, und
(iv) eine innere Schutzschicht über dem Kern (i), die von der Trennschicht (ii) verschieden ist, sich zwischen dem Kern und der Trennschicht (ii) befindet und einen Zucker aufweist.

2. Pharmazeutische Zubereitung nach Anspruch 1, wobei der Ester in der Trennschicht ein optional substituiertes Celluloseacetat, ein optional substituiertes Cellulosesuccinat oder ein optional substituiertes Celluloseacetatsuccinat ist, insbesondere Hydroxypropylmethylcelluloseacetatsuccinat.

3. Pharmazeutische Zubereitung nach Anspruch 1 oder 2, wobei das (Meth)acrylsäure-haltige Polymer in der magensaftresistenten Schicht ein Methacrylsäure-Alkylacrylat ist, insbesondere Methacrylsäure-Ethylacrylat.

4. Pharmazeutische Zubereitung nach einem der Ansprüche 1 bis 3, in welcher die innere Schutzschicht Rohrzucker, sowie optional ein wasserlösliches Polymer wie etwa Hydroxypropylmethylcellulose aufweist.

5. Pharmazeutische Zubereitung nach einem der Ansprüche 1 bis 4, wobei die magensaftresistente Schicht kein Hydroxypropylmethylcelluloseacetatsuccinat enthält.

6. Pharmazeutische Zubereitung nach einem der Ansprüche 1 bis 5, wobei die magensaftresistente Schicht einen Anteil an der Zusammensetzung von ≤ 20 Gew.-%, bevorzugt ≤ 16 Gew.-%, mehr bevorzugt ≤ 13 Gew.-% und insbesondere ≤ 10 Gew.-% hat.

7. Pharmazeutische Zubereitung nach einem der Ansprüche 1 bis 6, wobei die magensaftresistente Schicht so ausgebildet ist, dass sechs Proben der Zubereitung über zwei Stunden in 750 ml 0,1 N HCl bei 37 ± 0,5 °C im Drehkörbchen bei 100 U/min eine Freisetzung von Duloxetinhydrochlorid von ≤ 10 % aufweisen.

8. Pharmazeutische Zubereitung nach einem der Ansprüche 1 bis 7, wobei die Trennschicht so ausgebildet ist, dass bei fehlender magensaftresistenter Schicht sechs Proben der Zubereitung über zwei Stunden in 750 ml 0,1 N HCl bei 37 ± 0,5 °C im Drehkörbchen bei 100 U/min eine Freisetzung von Duloxetinhydrochlorid von > 10 % bei mindestens einer der sechs Zubereitungen aufweisen.

9. Pharmazeutische Zubereitung nach einem der Ansprüche 1 bis 8, wobei eine Probe in 1000 ml Acetatpufferlösung mit einem pH-Wert von 5,5 bei 37 ± 0,5 °C im Drehkörbchen bei 100 U/min eine Freisetzung von ≥ 90 % des Wirkstoffes nach einem Zeitraum von ≤ 7 Stunden, bevorzugt ≤ 5 Stunden, mehr bevorzugt ≤ 4 Stunden und insbesondere ≤ 3 Stunden aufweist.

10. Pharmazeutische Zubereitung nach einem der Ansprüche 1 bis 9, wobei der Wirkstoff aufweisende Kern aus einem wirkstofffreien inerten inneren Kern sowie einer Wirkstoffschicht über dem inneren Kern besteht, wobei die Wirkstoffschicht Duloxetin oder ein pharmazeutisch annehmbares Salz davon, insbesondere Duloxetinhydrochlorid, umfasst.

11. Pharmazeutische Zubereitung nach einem der Ansprüche 1 bis 9, wobei der Wirkstoff aufweisende Kern aus einem Matrixmaterial besteht, in welches Duloxetin oder ein pharmazeutisch annehmbares Salz davon, insbesondere Duloxetinhydrochlorid, eingebettet ist.

12. Magensaftresistente Duloxetinhydrochloridformulierung in Pelletform mit folgendem Pelletaufbau:
(i) einem inerten Kern,
(ii) einer Wirkstoffschicht über dem Kern, welche Duloxetinhydrochlorid, Hydroxypropylmethylcellulose und Talkum aufweist,
(iii) einer inneren Schutzschicht über der Wirkstoffschicht, welche Hydroxypropylmethylcellulose, Rohrzucker und Talkum aufweist,
(iv) einer von der inneren Schutzschicht verschiedenen Trennschicht über der inneren Schutzschicht, welche Hydroxypropylmethylcelluloseacetatsuccinat, Talkum und Triethylcitrat aufweist, und
(v) einer von der inneren Schutzschicht und der Trennschicht verschiedenen, magensaftresistenten Schicht über der Trennschicht, welche Methacrylsäure-Ethylacrylat-Copolymer, Triethylcitrat, Talkum und Natriumhydroxid aufweist.

13. Magensaftresistente Duloxetinhydrochloridformulierung in Pelletform mit folgendem Pelletaufbau:
(i) einem Kern bestehend aus Duloxetinhydrochlorid in einer Matrix aus mikrokristalliner Cellulose, quervernetztem Polyvinylpyrrolidon und Mannitol,
(ii) einer inneren Schutzschicht über der Wirkstoffschicht, welche Hydroxypropylmethylcellulose, Rohrzucker und Talkum aufweist,
(iii) einer von der inneren Schutzschicht verschiedenen Trennschicht über der inneren Schutzschicht, welche Hydroxypropylmethylcelluloseacetatsuccinat, Talkum und Triethylcitrat aufweist, und
(iv) einer von der inneren Schutzschicht und der Trennschicht verschiedenen, magensaftresistenten Schicht über der Trennschicht, welche Methacrylsäure-Ethylacrylat-Copolymer, Triethylcitrat, Talkum und Natriumhydroxid aufweist.

14. Darreichungsform enthaltend eine pharmazeutische Zubereitung nach einem der Ansprüche 1 bis 11 oder eine magensaftresistente Duloxetinhydrochloridformulierung nach Anspruch 12 oder 13, wobei die Darreichungsform eine Tablette oder eine Kapsel ist, bevorzugt eine Kapsel und mehr bevorzugt eine Hartkapsel.

## Claims

1. Pharmaceutical preparation in pellet form comprising
(i) an active substance-containing core, comprising duloxetine or a pharmaceutically acceptable salt thereof, especially duloxetine hydrochloride,
(ii) a separating layer comprising an ester of cellulose optionally substituted with one or several organic acids,
(iii) an enteric layer differing from the separating layer comprising a (meth)acrylic acid-containing polymer, wherein the separating layer (ii) is between the core (i) and the enteric layer (iii), and
(iv) an inner protective layer coating the core (i) and differing from the separating layer located between the core and the separating layer and comprising a sugar.

2. Pharmaceutical preparation according to claim 1, wherein the ester in the separating layer is an optionally substituted cellulose acetate, an optionally substituted cellulose succinate or an optionally substituted cellulose acetate succinate, especially hydroxypropyl methylcellulose acetate succinate.

3. Pharmaceutical preparation according to claim 1 or 2, wherein the (meth)acrylic acid-containing polymer in the enteric layer is a methacrylic acid alkylacrylate, especially methacrylic acid ethylacrylate.

4. Pharmaceutical preparation according to one of the claims 1 to 3, wherein the inner protective layer comprises cane sugar, as well as optionally a water-soluble polymer such as hydroxypropyl methylcellulose.

5. Pharmaceutical preparation according to one of the claims 1 to 4, wherein the enteric layer does not comprise hydroxypropyl methylcellulose acetate succinate.

6. Pharmaceutical preparation according to one of the claims 1 to 5, wherein the enteric layer makes up ≤ 20 wt.-%, preferably ≤ 16 wt.-%, more preferably ≤ 13 wt.- % and especially ≤ 10 wt.-% of the composition.

7. Pharmaceutical preparation according to one of the claims 1 to 6, wherein the enteric layer is formed in such a way that six samples of the preparation over a period of two hours in 750 ml 0.1 N HCl at 37 ± 0.5 °C in a rotating basket at 100 rpm have a release rate of duloxetine hydrochloride of ≤ 10 %.

8. Pharmaceutical preparation according to one of the claims 1 to 7, wherein the separating layer is formed in such a way that in case of a missing enteric layer at least one out of six samples of the preparation over a period of two hours in 750 ml 0.1 N HCl at 37 ± 0.5 °C in a rotating basket at 100 rpm has a release rate of duloxetine hydrochloride of > 10 %.

9. Pharmaceutical preparation according to one of the claims 1 to 8, wherein one sample in 1000 ml acetate buffer solution with a pH-value of 5.5 at 37 ± 0.5 °C in a rotating basket at 100 rpm has a release rate of ≥ 90 % of the active substance after ≤ 7 hours, preferably ≤ 5 hours, more preferably ≤ 4 hours and especially ≤ 3 hours.

10. Pharmaceutical preparation according to one of the claims 1 to 9, wherein the active substance-containing core consists of a no active substance-comprising inert inner core as well as an active substance layer coating the inner core, wherein the active substance layer comprises duloxetine or a pharmaceutically acceptable salt thereof, especially duloxetine hydrochloride.

11. Pharmaceutical preparation according to one of the claims 1 to 9, wherein the active substance-containing core consists of a matrix material into which duloxetine or a pharmaceutically acceptable salt thereof, especially duloxetine hydrochloride, is embedded.

12. Enteric duloxetine hydrochloride formulation in pellet form, the pellet having the following structure:
(i) an inert core,
(ii) an active substance layer coating the core, containing duloxetine hydrochloride, hydroxypropyl methylcellulose and talcum,
(iii) an inner protective layer coating the active substance layer containing hydroxypropyl methylcellulose, cane sugar and talcum,
(iv) a separating layer differing from the inner protective layer coating the inner protective layer with hydroxypropyl methylcellulose acetate succinate, talcum and triethyl citrate, and
(v) an enteric layer differing from the inner protective layer and the separating layer coating the separating layer containing a methacrylic acid ethylacrylate copolymer, triethyl citrate, talcum and sodium hydroxide.

13. Enteric duloxetine hydrochloride formulation in pellet form, the pellet having the following structure:
(i) a core consisting of duloxetine hydrochloride in a matrix made of microcrystalline cellulose, cross-linked polyvinylpyrrolidone and mannitol,
(ii) an inner protective layer coating the active substance layer containing hydroxypropyl methylcellulose, cane sugar and talcum,
(iii) a separating layer differing from the inner protective layer containing hydroxypropyl methylcellulose acetate succinate, talcum and triethyl citrate, and
(v) an enteric layer differing from the inner protective layer and the separating layer coating the separating layer containing a methacrylic acid-containing polymer, triethyl citrate, talcum and sodium hydroxide.

14. Dosage form comprising a pharmaceutical preparation according to one of the claims 1 to 11 or an enteric duloxetine hydrochloride formulation according to claim 12 or 13, wherein the dosage form is a tablet or capsule, preferably a tablet and more preferably a hard capsule.

## Revendications

1. Préparation pharmaceutique sous forme de pellet, comprenant
(i) un noyau présentant un principe actif comprenant de la duloxétine ou un sel pharmaceutiquement acceptable de celle-ci, notamment de l'hydrochlorure de duloxétine,
(ii) une couche de séparation, comprenant de l'ester de cellulose facultativement substitué avec une ou plusieurs acides organiques,
(iii) une couche entérique autre que la couche de séparation comprenant un polymère contenant de l'acide (méth)acrylique, la couche de séparation (ii) étant située entre le noyau (i) et la couche entérique (iii), et
(iv) une couche de protection intérieure au-dessus du noyau (i) autre que la couche de séparation (ii) disposée entre le noyau et la couche de séparation (iii) et présentant un sucre.

2. Préparation pharmaceutique selon la revendication 1, dans laquelle l'ester contenu dans la couche de séparation est un acétate de cellulose facultativement substitué, un succinate de cellulose facultativement substitué ou un succinate d'acétate de cellulose facultativement substitué, en particulier le succinate d'acétate d'hydroxypropylméthylcellulose.

3. Préparation pharmaceutique selon la revendication 1 ou 2, dans laquelle le polymère contenant l'acide (méth)acrylique contenu dans la couche entérique est un acrylate d'alkyle d'acide méthacrylique, notamment un acrylate d'éthyle d'acide méthacrylique.

4. Préparation pharmaceutique selon l'une des revendications 1 à 3, dans laquelle la couche de protection intérieure contient du sucre de canne ainsi que, de manière facultative, un polymère soluble dans l'eau tel que par exemple l'hydroxypropylméthylcellulose.

5. Préparation pharmaceutique selon l'une des revendications 1 à 4, dans laquelle la couche entérique ne contient pas de succinate d'acétate d'hydroxypropylméthylcellulose.

6. Préparation pharmaceutique selon l'une des revendications 1 à 5, dans laquelle la couche entérique présente une proportion de la composition de ≤ 20 % en poids, de préférence ≤ 16 % en poids, de préférence encore ≤ 13 % en poids en notamment ≤ 10 % en poids.

7. Préparation pharmaceutique selon l'une des revendications 1 à 6, dans laquelle la couche entérique est prévue de manière que six échantillons de la préparation présentent, maintenus pendant deux heures dans 750 ml de 0,1 N HCI, à 37 ± 0,5°C dans un panier tournant à 100 tr/min, une libération d'hydrochlorure de duloxétine de ≤ 10 %.

8. Préparation pharmaceutique selon l'une des revendications 1 à 7, dans laquelle la couche de séparation est prévue de manière qu'en absence de couche entérique, six échantillons de la préparation présentent, maintenus pendant deux heures dans 750 ml de 0,1 N HCI, à 37 ± 0,5°C dans un panier tournant à 100 tr/min, une libération d'hydrochlorure de duloxétine de > 10 %, sur une au moins des six préparations.

9. Préparation pharmaceutique selon l'une des revendications 1 à 8, dans laquelle un échantillon maintenu dans une solution de 1000 ml d'un tampon à base d'acétate ayant une valeur pH de 5,5 à 37 ± 0,5°C dans un panier tournant à 100 tr/min, présente une libération de ≥ 90 % du principe actif après une durée de ≤ 7 heures, de préférence ≤ 5 heures, de préférence encore ≤ 4 heures et notamment ≤ 3 heures.

10. Préparation pharmaceutique selon l'une des revendications 1 à 9, dans laquelle le noyau présentant le principe actif consiste en un noyau intérieur inerte exempt de principe actif ainsi qu'en une couche de principe actif au-dessus du noyau intérieur, la couche de principe actif contenant de la duloxétine ou un sel pharmaceutiquement acceptable de celle-ci, en particulier de l'hydrochlorure de duloxétine.

11. Préparation pharmaceutique selon l'une des revendications 1 à 9, dans laquelle le noyau présentant du principe actif est constitué par un matériau matriciel dans lequel est incorporé de la duloxétine ou un sel pharmaceutiquement acceptable de celle-ci, en particulier de l'hydrochlorure de duloxétine.

12. Formulation entérique d'hydrochlorure de duloxétine sous forme de pellet, comprenant la structure de pellet suivante:
(i) un noyau inerte,
(ii) une couche de principe actif au-dessus du noyau, contenant de l'hydrochlorure de duloxétine, de l'hydroxypropylméthylcellulose et du talc,
(iii) une couche de protection intérieure au-dessus de la couche de principe actif, qui contient de l'hydroxypropylméthylcellulose, du sucre de canne et du talc,
(iv) une couche de séparation autre que la couche de protection intérieure prévue au-dessus de la couche de protection intérieure qui contient du succinate d'acétate d'hydroxypropylméthylcellulose, du talc et du citrate de triéthyle, et
(v) une couche entérique autre que la couche de protection intérieure et autre que la couche de séparation prévue au-dessus de la couche de séparation qui contient du copolymère d'acide méthacrylique-acrylate d'éthyle, du citrate de triéthyle, du talc et de l'hydroxyde de sodium.

13. Formulation entérique d'hydrochlorure de duloxétine sous forme de pellet, comprenant la structure de pellet suivante:
(i) un noyau constitué par de l'hydrochlorure de duloxétine dans une matrice de cellulose microcristalline, de polyvinylpyrrolidone réticulée et de mannitol,
(ii) une couche de protection intérieure au-dessus de la couche de principe actif, qui contient de l'hydroxypropylméthylcellulose, du sucre de canne et du talc,
(iii) une couche de séparation autre que la couche de protection intérieure prévue au-dessus de la couche de protection intérieure qui contient du succinate d'acétate d'hydroxypropylméthylcellulose, du talc et du citrate de triéthyle, et
(iv) une couche entérique autre que la couche de protection intérieure et autre que la couche de séparation prévue au-dessus de la couche de séparation qui contient du copolymère d'acide méthacrylique-acrylate d'éthyle, du citrate de triéthyle, du talc et de l'hydroxyde de sodium.

14. Forme d'administration contenant une préparation pharmaceutique selon l'une des revendications 1 à 11 ou une formulation entérique d'hydrochlorure de duloxétine selon la revendication 12 ou 13, la forme d'administration étant un comprimé ou une capsule, de préférence une capsule et de préférence encore une capsule dure.
